# EUROPEAN PATENT APPLICATION

(11) **EP 2 759 541 A1**
(43) Date of publication of application: **30.07.2014**
(21) Application number: 12885183.9
(22) Date of filing: 25.10.2012
(51) Int. Cl.: C07D 307/06

(54) **CONTINUOUS PRODUCTION METHOD OF 2-METHF**

(71) Applicant: Asymchem Laboratories (Tianjin) Co., Ltd, Tianjin 300457 (CN); Asymchem Life Science (Tianjin) Co., Ltd, Tianjin 300457 (CH); Tianjin Asymchem Pharmaceutical Co., Ltd, Tianjin 300000 (CN); Asymchem Laboratories (Fuxin) Co., Ltd, Fuxin, Liaoning 123000 (CN); Jilin Asymchem Laboratories Co., Ltd, Dunhua, Jilin 133700 (CN)
(72) Inventor: HONG, Hao, Tianjin 300457 (CN); HUANG, Pingzhong, Tianjin 300457 (CN); LU, Jiangping, Tianjin 300457 (CN); ZHANG, Xin, Tianjin 300457 (CN); GUO, Xiaowen, Tianjin 300457 (CN); SUN, Xingfang, Tianjin 300457 (CN); TAO, Jian, Tianjin 300457 (CN)
(74) Representative: Müller-Boré & Partner Patentanwälte PartG mbB
(86) International application number: PCT/CN2012/083522
(87) International publication number: WO 2014/063333

(57) **Abstract**

The disclosure claims a continuous processing method of 2-Methyltetrahydrofuran (2-MeTHF), including the steps as follows: introducing gasification furfural and hydrogen into a first reaction zone and processing a first catalytic hydrogenation reaction; introducing the gas which is output from the first reaction zone to a second reaction zone to implement a secondary catalytic hydrogenation reaction; and condensing the gas output from the second reaction zone to obtain the 2-MeTNF; wherein, the first reaction zone is filled with a catalyst which is used for aldehyde group reduction, and the second reaction zone is filled with the catalyst for aromatic saturation hydrogenation. By adopting the low-toxicity catalyst which is cheap and easy to get, the high-purity 2-MeTHF can be produced by implementing the gas-phase continuous reaction by the furfural under low pressure or ambient pressure, the traditional process which has high pressure, high investment and high risk can be changed, and use of the high- toxicity noble metal catalyst can be reduced. The method provided by the disclosure has simple processing process, low investment, low risk, high throughput and high yield of furfural in unit time; the crude product obtained by the method has high purity, and the impurities can be easily separated.

## Description

### Technical field of the invention

The disclosure relates to the field of chemical synthesis techniques, and in particular to a continuous processing method of 2-Methyltetrahydrofuran.

### Background of the invention

The 2-Methyltetrahydrofuran (2-MeTHF) is an important organic intermediate and an excellent solvent; as it has moderate boiling point (80.2 degrees centigrade) and low solubility in water, is easy to be separated from the water, and synchronously has the properties of Louis alkaline similar with Tetrahydrofuran (THF), the 2-MeTHF can be applied to various organometallic reactions, and now the 2-MeTHF is widely applied in the industrial production as a new solvent. As the 2-MeTHF can be mutually soluble with gasoline in any proportion, and has excellent properties, such as oxidation, vapor pressure and the like, the 2-MeTHF can be used as the automobile fuel additives to replace part of the gasoline. Via research, the engine performance cannot be influenced at all when the proportion of the 2-MeTHF in the gasoline is greater than 60%, and the fuel consumption of the automobiles also cannot be increased. In addition, the 2-MeTHF is also the raw material of the pharmaceutical industry, and can be used for synthesis of the anti-hemorrhoid drugs such as primaquine diphosphate and the like.

Furfural is the starting raw material for producing the 2-MeTHF, and is produced by hydrolyzing and dehydrating the agricultural and sideline products such as ear of corn, bagasse and the like, belonging to the renewable raw materials. Compared with the method for extracting the 2-MeTHF from petroleum, the method for synthesizing the 2-MeTHF by taking the furfural as the starting raw material has advantages of lower cost, more clean, and sustainable development.

At present, the method for producing the 2-MeTHF by using the furfural as the starting raw material needs to implement two steps of catalytic hydrogenation reactions, namely, firstly using the furfural to produce 2-Methyl Furan (2-MeF) via continuous gas-phase reaction, and then using the 2-MeF to produce the 2-MeTHF via high-pressure catalytic hydrogenation. During the reaction of using the 2-MeF to produce the 2-MeTHF via catalytic hydrogenation, the 2-MeF will be reduced to the 2-MeTHF at 150 degrees centigrade and 20-15MPa pressure. In the present industrial production, the above routing processes need the separation and purification of the intermediate product 2-MeF, particularly, the second step needs higher pressure, harsh reaction conditions and high equipment investment.

Proskuryakov et al. use the furfural as the starting raw material to directly prepare the 2-MeTHF in an autoclave at 220 degrees centigrade and 160atm, wherein the proportion of the mixed catalyst copper chromite and Raney Ni is 1:1; the yield of directly converting the furfural to the 2-MeTHF under such conditions is very low, even not greater than 42%.

Except low furfural conversion rate, the prior art also has the following disadvantages: 1) the liquid-phase high-pressure process of the 2-MeTHF has high reaction pressure, high risk, and needs to implement the separation of the intermediate product, which causes high investment; 2) because of the using of continuous gas-phase reaction, the handling capability of raw material is small , or the catalyst containing high-toxicity chromium will be used. The above problems cause high cost, which greatly influence the industrial amplification of the process. Thus, how to solve the present technical problems that the process of the 2-MeTHF has over-high pressure, high catalyst toxicity and high cost, needs to implement the separation and purification for the intermediate products, and cannot be continuously processing has become the current research hotspot.

### Summary of the invention

The disclosure aims at providing a continuous processing method of 2-MeTHF, which can continuously produce the high-purity 2-MeTHF by using a non-toxic catalyst under ambient pressure or low pressure.

In order to realize the above purpose, the disclosure provides a continuous processing method of the 2-MeTHF according to one aspect, including the following steps: introducing gasification furfural and hydrogen into a first reaction zone and processing a first catalytic hydrogenation reaction; introducing the gas output from the first reaction zone into a second reaction zone and processing a secondary catalytic hydrogenation reaction; and condensing the gas output from the second reaction zone to obtain the 2-MeTHF; wherein, the first reaction zone is filled with a catalyst which is used for aldehyde group reduction, and the second reaction zone is filled with the catalyst which is used for aromatic saturation hydrogenation.

Further, before introducing the gasification furfural and the hydrogen into the first reaction zone, further includes a step of premixing the gasification furfural and the hydrogen.

Further, before the secondary catalytic hydrogenation reaction, further includes a step of implementing heat exchange for the gas output from the first reaction zone.

Further, the catalyst used for aldehyde group reduction is the copper-based catalyst; and the catalyst used for the aromatic saturation hydrogenation is the nickel-based catalyst.

Further, in the first reaction zone, the flow of the gasification furfural is 0.05-2.0Kg/(Kg•h) based on the weight of the copper-based catalyst, and preferably is 0.1-1.0Kg/(Kg•h); in the second reaction zone, the flow of the gasification furfural is 0.05-2.0Kg/(Kg•h) based on the weight of the nickel-based catalyst, and preferably is 0.1-1.0Kg/(Kg•h).

Further, in the first reaction zone and the second reaction zone, the molar ratio of the hydrogen flow and the gasification furfural flow is greater than 4:1, preferably, the molar ratio is 5:1 to 100:1, and more preferably, the molar ratio is 7:1 to 40:1.

Further, providing a desulfurization catalyst and/or a carbon monoxide (CO) conversion catalyst to the first reaction zone and/or the second reaction zone.

Further, the method includes: pumping the liquid furfural into a gasification chamber via a charging pump, and gasifying the furfural to obtain the gasification furfural; premixing the gasification furfural and the hydrogen in the gasification chamber, and inputting the mixed gas to a first hydrogenation fixed bed reactor and processing a first catalytic hydrogenation reaction; introducing the gas output from the first hydrogenation fixed bed reactor into a heat exchange device to implement heat exchange; introducing the gas output from the heat exchange deice into a second hydrogenation fixed bed reactor and processing a secondary catalytic hydrogenation reaction; and introducing the gas output from the second hydrogenation fixed bed reactor into a condensing unit for condensation to obtain the 2-MeTHF.

Further, the hot-spot temperature of the catalyst bed layer of the first hydrogenation fixed bed reactor is 180 degrees centigrade to 300 degrees centigrade, and preferably is 180 degrees centigrade to 230 degrees centigrade; the hot-spot temperature of the catalyst bed layer of the second hydrogenation fixed bed reactor is 80 degrees centigrade to 180 degrees centigrade, and preferably is 80 degrees centigrade to 130 degrees centigrade.

Further, both the first hydrogenation fixed bed reactor and the second hydrogenation fixed bed reactor are the tubular fixed bed reactors or multitubular fixed bed reactors.

The disclosure adopts the low-toxicity catalyst which is cheap and easy to get, and uses the furfural to produce the high-purity 2-MeTHF via the gas-phase continuous reaction under low pressure or ambient pressure, changes the traditional process of producing the 2-MeTHF which has high pressure, high investment and high risk, and reduces the use of the high-toxicity and high-cost catalysts in the prior art. The method has the advantages of low investment, low risk, high throughput and high yield of furfural in unit time, simple process and the like, and reduces the use of the toxic catalyst and the high-cost noble metals during the process; in the obtained crude products, the target product 2-MeTHF has high purity, and the impurities are easy to be separated, thus the method is suitable for the industrial production.

### Brief description of the drawings

The drawing which form a part of the application is used for further understanding the disclosure; the exemplary embodiments of the disclosure and the descriptions thereof are used for explaining the disclosure, without improperly limiting the disclosure. In the drawings:

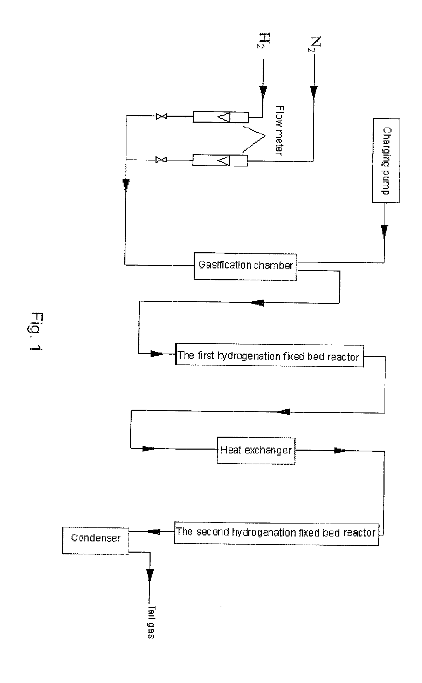

Fig. 1 shows a process flowchart for continuous process of 2-MeTHF in a typical embodiment of the disclosure.

### Detailed description of the embodiments

It should note that, the embodiments of the application and the characteristics in the embodiments can be mutually combined without conflict. The disclosure is described below with reference to the drawings and embodiments in details.

According to a typical embodiment of the disclosure, the continuous processing method of 2-MeTHF includes the steps of: introducing gasification furfural and hydrogen into a first reaction zone and processing a first catalytic hydrogenation reaction; introducing the gas output from the first reaction zone into a second reaction zone and processing a secondary catalytic hydrogenation reaction; and condensing the gas output from the second reaction zone to obtain the 2-MeTHF; wherein, the first reaction zone is filled with a catalyst which is used for aldehyde group reduction, and the second reaction zone is filled with the catalyst which is used for aromatic saturation hydrogenation.

The disclosure adopts the low-toxicity catalyst which is cheap and easy to get, and uses the furfural to produce the high-purity 2-MeTHF via the gas-phase continuous reaction under low pressure or ambient pressure, changes the traditional process of producing the 2-MeTHF which has high pressure, high investment and high risk, and reduces the use of the high-toxicity and high-cost catalysts in the prior art. The method has the advantages of low investment, low risk, high throughput and high yield of furfural in unit time, simple process and the like, and reduces the use of the toxic catalyst and the high-cost noble metals during the process; in the obtained crude products, the target product 2-MeTHF has high purity, and the impurities are easy to be separated, thus the method is suitable for the industrial production.

According to a typical embodiment of the disclosure, before introducing the gasification furfural and the hydrogen into the first reaction zone, further includes a step of premixing the gasification furfural and the hydrogen. The commercial furfural adopted by the disclosure is a liquid raw material, and the liquid raw material is fully mixed with gaseous hydrogen after being gasified, so as to guarantee that the mixed gas can be fully reacted under the effect of the catalysts after entering into the first reaction zone, thus improving the conversion rate.

According to another typical embodiment of the disclosure, before the secondary catalytic hydrogenation reaction, further includes a step of implementing heat exchange for the gas output from the first reaction zone. As the first catalytic hydrogenation reaction of the furfural is a violent exothermic reaction, and the main components obtained from the reaction are the mixed gas of the 2-MeF and the hydrogen; as the mixed gas has high temperature, the total yield can be reduced due to the decreased reaction selectivity if processing the secondary catalytic hydrogenation reaction at such temperature, heat exchange is implemented for the mixed gas of the 2-MeF and the hydrogen between the first reaction zone and the second reaction zone to guarantee the suitable inlet temperature before the secondary catalytic hydrogenation reaction. In addition, the impurities with high boiling points can be removed during the heat exchange, which is beneficial for processing the secondary catalytic hydrogenation reaction.

Preferably, the catalyst for aldehyde group reduction adopted by the disclosure is the copper-based catalyst; and the catalyst used for the aromatic saturation hydrogenation is the nickel-based catalyst. Preferably, the copper-based catalyst includes TG-45 and/or GC207; and the nickel-based catalyst includes RTH-2123E and/or HT-40. The disclosure preferably selects, but is not limited to the above catalysts; and all the above catalysts are commercial products, which are cheap and easy to get, and have low toxicity.

According to a typical embodiment of the disclosure, as shown in Fig. 1, the processing method of preparing 2-MeTHF via furfural includes the steps of: pumping the liquid furfural into a gasification chamber via a charging pump, and gasifying the furfural; premixing the gasification furfural and the hydrogen in the gasification chamber, and inputting the mixed gas to a first hydrogenation fixed bed reactor and processing a first catalytic hydrogenation reaction; introducing the gas output from the first hydrogenation fixed bed reactor into a heat exchange device; introducing the gas output from the heat exchange deice into a second hydrogenation fixed bed reactor and processing a secondary catalytic hydrogenation reaction; and introducing the gas output from the second hydrogenation fixed bed reactor into a condensing unit for condensation to obtain the 2-MeTHF.

The process of the 2-MeTHF by adopting the furfural via the catalytic hydrogenation gas-phase continuous reaction under the ambient pressure includes the devices, such as the gasification chamber, the first hydrogenation fixed bed reactor, the heat exchange device, the second hydrogenation fixed bed reactor and the condensing unit and the like, according to the sequence of connection.

The reaction process of preparing the 2-MeTHF via the furfural mainly includes the steps that: the liquid furfural is pumped into the gasification chamber via the charging pump, is gasified in the gasification chamber, and is premixed with excess hydrogen to form the first-level feed gas; the first-level feed gas is input to the first hydrogenation fixed bed reactor which is filled with the copper-based catalysts to process the first catalytic hydrogenation reaction, so as to obtain the second-level feed gas, of which the main components are 2-MeF and the hydrogen. The second-level feed gas is input to the heat exchange device to implement heat exchange, and meanwhile, the impurities with high boiling points are removed. The second-level feed gas which is implemented with heat exchange is input to the second hydrogenation fixed bed reactor which is filled with the nickel-based catalyst to process the secondary catalytic hydrogenation reaction; the crude mixed gas obtained from the reaction is input to the condensing unit for condensation; the final gas is cooled to be 0-10 degrees centigrade via the condensing unit; the gas phase and the liquid phase are formed after condensation; the main component of the liquid from the condensing unit is the 2-MeTHF, which is divided into two layers after standing, namely, an organic phase and an aqueous phase; the lower-layer phase includes more than 90% of water; and except the 2-MeTHF, the upper-layer phase only includes a small amount of by-products which can be removed by any subsequent purification and distillation.

The gas (which is mainly the excess hydrogen) from the condensing unit is input to a gas compressor for recycling according to a certain proportion, or is directly removed, or is burnt by an incinerator. Preferably, the gas is introduced into the reactor again circularly, so as to increase the use ratio of the hydrogen. The excess hydrogen is introduced during the whole reaction process to guarantee that the furfural is fully converted to the 2-MeF, and the 2-MeF is fully converted to the 2-MeTHF, thus improving the conversion rate. The heat exchange device in the disclosure is preferably a heat exchanger, and the condensing unit is preferably a condenser.

The gasification chamber and the condenser adopted by the disclosure can be designed to have various types and specifications as required, aiming to guarantee the effective gasification of the raw material furfural and the effective condensation of the final product 2-MeTHF. The heat exchanger is arranged between the first hydrogenation fixed bed reactor and the second hydrogenation fixed bed reactor, wherein the heat changer can have various types and specifications, aiming to implement effective temperature control for the gas which is entering into the second hydrogenation fixed bed reactor, and to remove the impurities with high boiling point which can be condensed in the second hydrogenation fixed bed reactor, so as to influence the catalyst activity.

The first hydrogenation fixed bed reactor is arranged in the first reaction zone, and the second hydrogenation fixed bed reactor is arranged in the second reaction zone, wherein the first hydrogenation fixed bed reactor is filled with the copper-based catalyst. As some existing catalysts, such as the copper-chrome catalyst series, greatly influence the environment, although the catalysts have high activity and selectivity in process of the 2-MeF via the catalytic hydrogenation reaction of the furfural, as the chrome composites have high toxicity which brings serious environment pollution problems for the production and catalyst recycling, the disclosure abandons some conventional catalysts, preferably selects the non-chrome copper-based catalyst which is cheap and environment friendly in the case of producing the 2-MeF by dehydrating the furfural, such as article number TG-45 (produced by Shanghai Xunkai Chemical technology Co., Ltd.) and article number GC207 (produced by Liaoning Haitai Science and Technology Development Co., Ltd.).

The mixed gas which is output after being implemented with the first catalytic hydrogenation reaction of the first hydrogenation fixed bed reactor is introduced into the second hydrogenation fixed bed reactor via the heat exchange device, and the second hydrogenation fixed bed reactor is filled with the nickel-based catalyst. Preferably, the alloy, framework, load and other types are adopted. The nickel-based catalyst, such as the article number RTH-2123E (produced by Dalian General Chemical Co., Ltd.) and HT-40 (produced by Liaoning Haitai Science and Technology Development Co., Ltd.), is adopted; wherein RTH-2123E is an irregular granular Raney Ni catalyst, and the HT-40 is a cylindrical load nickel catalyst.

Except providing the copper-based catalysts and the nickel-based catalyst of the catalytic hydrogenation reaction, the disclosure can also fill the catalysts with other functions on the inlet or outlet of the catalyst bed layer of the first fixed bed reactor, or the inlet of the catalyst bed layer of the second fixed bed reactor. Preferably, the desulfurization catalyst and/or CO conversion catalyst are/is also set in the first reaction zone and/or the second reaction zone. The catalysts can improve the quality of the products by removing the impurities of the raw material or converting the by-products, for example, the small amount of sulphur-containing components contained in the raw material furfural can be removed via the desulfurization catalyst; the reaction of producing the 2-MeF via furfural hydrogenation generally contains a small amount of CO, which can form the high-toxicity and volatile Ni(CO)₄ with the nickel, and the Ni(CO)4 can be converted to methanol by setting copper and/or ruthenium catalyst to implement hydrogenation reaction, so as to be removed.

Preferably, both the first hydrogenation fixed bed reactor and the second hydrogenation fixed bed reactor are the tubular fixed bed reactors or multitubular fixed bed reactors. The tubular fixed bed reactors are the multitubular fixed bed reactors after being amplified. The catalytic hydrogenation of the furfural and the catalytic hydrogenation of 2-MeF are both violent exothermic reactions, thus two sections of the fixed bed reactors are required for effective temperature control. The disclosure preferably adopts the tubular fixed bed reactor with a jacket, and uses cold oil to implement effective temperature control for the catalyst bed layer via the reactor jacket, and sets at least two temperature measuring points on the upper part and lower part of the catalyst bed layer, so as to monitor the hot-spot temperature of the catalyst bed layer randomly. The first fixed bed reactor and the second fixed bed reactor can have the same structures and catalyst filling solutions.

Further preferably, the stainless steel porous tray is arranged at the bottom of the tubular fixed bed reactor; and the upper part of the porous tray is sequentially filled with the inert filler, the catalyst and the inert filler. The selectable inert filler includes quartz sand, glass balls and the like. The filling amount of the catalysts for the two sections of hydrogenation reactors needs to be matched, for example, if selecting the catalyst combination of TG-45+RTH-2123E, in the TG-45 hydrogenation reaction section, the Weight Hourly Space Velocity (WHSV) of the catalyst of furfural can achieve 1.2Kg/(Kg•h), but in the RTH-2123E hydrogenation reaction section, the WHSV of the catalyst of furfural is 0.53Kg/(Kg•h), and this requires relatively small amount of TG-45 and relatively large amount of RTH-2123E. The feed gas can be introduced from the bottom of the fixed bed reactor, and can be discharged from the fixed bed reactor from the upper part after introducing into the catalyst bed layer, and also can be introduced from the upper part of the fixed bed reactor, and can be discharged from the fixed bed reactor from the lower part.

According to a typical embodiment of the disclosure, the hot-spot temperature of the catalyst bed layer of the first hydrogenation fixed bed reactor is 180 degrees centigrade to 300 degrees centigrade, and preferably is 180 degrees centigrade to 230 degrees centigrade; the hot-spot temperature of the catalyst bed layer of the second hydrogenation fixed bed reactor is 80 degrees centigrade to 180 degrees centigrade, and preferably is 80 degrees centigrade to 130 degrees centigrade.

The process of 2-MeTHF by furfural catalytic hydrogenation is the gas-phase continuous reaction under low pressure or the ambient pressure; the liquid furfural is introduced into the gasification chamber via the charging pump, the furfural is gasified and is premixed with the hydrogen, the temperature of the gasification temperature is 180-210 degrees centigrade, however, the hot-spot temperature of the catalyst bed layer of the first hydrogenation fixed bed reactor is kept at 180-300 degrees centigrade, on the basis of satisfying the projected output, in order to prolong the service life of the catalyst, the catalyst bed layer should be operated at lower temperature, preferably, the hot-spot temperature is kept at 180-230 degrees centigrade. The mixed gas output from the first hydrogenation fixed bed reactor is introduced into the heat exchanger, and the temperature of the heat exchanger is maintained at 65-80 degrees centigrade; the hot-spot temperature of the bed layer of the second hydrogenation fixed bed reactor is maintained at 80-180 degrees centigrade; in order to prolong the service life of the catalyst, the temperature of the catalyst bed layer is preferably controlled at 80-130 degrees centigrade.

The reason for controlling the temperature of the gasification chamber and the bed layer within the above range is further described below: the boiling point of the raw material liquid furfural is 162 degrees centigrade, and the temperature of the gasification chamber is 180-210 degrees centigrade, which can effectively gasify the liquid furfural; the furfural can be accelerated to be coked and carbonized in the gasification chamber if the temperature is too high; and the reaction selectivity can be weakened while the service life of the catalyst can be shortened if the temperature of the bed layer is too high, so, the better reaction effect can be obtained by controlling the temperature of the bed layer within the above range.

In the actual process in the future, the catalysts can be combined in order to achieve the expected yield, improve the product purity and guarantee the biggest benefits; the molar ratio of the hydrogen and the raw material furfural, the reaction temperature and the space velocity of the 2-MeF need to be comprehensively selected when combining.

According to a preferred embodiment of the disclosure, during the continuous process of the 2-MeTHF, in the first reaction zone, the flow of the gasification furfural is 0.05-2Kg/(Kg•h) based on the weight of the copper-based catalyst, and preferably is 0.1-1.0Kg/(Kg•h); in the second reaction zone, the flow of the gasification furfural is 0.05-2.0Kg/(Kg•h) based on the weight of the nickel-based catalyst, and preferably is 0.1-1.0Kg/(Kg•h).

Here, the flow calculation method of the furfural is that: the weight velocity (Kg/h) of the furfural divides the weight (Kg) of the copper-based catalyst or the nickel-based catalyst. If the furfural has over-high flow, the flow velocity can be too fast under the corresponding molar ratio of the hydrogen and raw material furfural and the temperature, so that the reaction is incomplete; if the flow velocity is too slow, the impurities caused by excessive hydrogenation can be generated. The hydrogen adopted by the disclosure is any gas containing the free hydrogen, but the gas cannot contain the harmful amount of catalyst toxicants, such as CO, sulphur-containing components, halogen and the like. The exhaust gas of a reformer can be adopted to achieve the purpose of waste recovery, preferably, the pure hydrogen is used as the hydrogenation gas.

According to a typical embodiment of the disclosure, in the first reaction zone and the second reaction zone, the molar ratio of the hydrogen flow and the flow of the gasification furfural is greater than 4:1; preferably, the molar ratio is 5:1 to 100:1; more preferably, the molar ratio is 7:1 to 40:1. By controlling the hydrogen flow within the above range, the furfural can be fully converted to the 2-MeTHF; and the waste of hydrogen cannot be caused.

The beneficial effects of the disclosure are further described below with reference to the embodiments:

### Embodiment 1

Reaction devices: the gasification chamber, the first hydrogenation fixed bed reactor (DN25 white steel pipe, with 50cm of length), the heat exchanger, the second hydrogenation fixed bed reactor (DN25 white steel pipe, with 50cm of length), and the condenser.

Reaction process:
(1) the first hydrogenation fixed bed reactor is filled with 100g of copper-based catalyst (of which the article number is TG-45, from Shanghai Xunkai), and the height of the catalyst bed after being filled is 15cm. The temperature measuring points are respectively set on the upper part and lower part of the catalyst bed layer of the first fixed bed reactor. Approximately 150g (of which the wet weight is 240g) of Raney Ni catalyst (of which the article number is RTH-2123E, form Dalian Tonghua) is filled in the second fixed bed reactor via a wet packing method; the height of the filled catalyst bed is about 15cm. And the temperature measuring points are respectively set on the upper part and lower part of the catalyst bed layer of the second fixed bed reactor.
(2) The gas flows through the first fixed bed reactor from top to bottom. The catalysts are activated by those skilled in the art to use the nitrogen/hydrogen mixture under the ambient pressure. In the mixed gas, the hydrogen concentration is slowly increased to 100% from 0. After the gas for reducing and activating the copper-based catalyst is discharged from the first fixed bed reactor, the gas is implemented with heat exchange to be 130 degrees centigrade in the heat exchanger, and then is introduced into the second fixed bed reactor to dry the Raney Ni catalyst.

After completing the reduction activation and drying for the catalyst, adjusting the temperature of the gasification chamber, the oil bath temperature of the first hydrogenation fixed bed reactor, the temperature of the heat exchanger and the oil bath temperature of the second hydrogenation fixed bed reactor, and synchronously adjusting the hydrogen flow; adjusting the liquid furfural flow to be 0.6mL/min. measuring the furfural conversion rate under the above condition, and the purity of the 2-MeTHF in the organic phase in the crude products.

### Embodiments 2-3

The operation steps are basically the same as the embodiment 1, and the difference is the catalyst type, reaction temperature, flow and the like, specifically shown in Table 1.

Wherein the reaction processes of the embodiments 1-3 are all under low pressure or ambient pressure, and the specific conditions and results are shown in Table 1.

**Table 1**

| Conditions | | Embodiment 1 | Embodiment 2 | Embodiment 3 |
|---|---|---|---|---|
| Reaction pressure | | Ambient pressure | Ambient pressure | 0.1MPa |
| Temperature of the gasification chamber (°C) | | 180 | 195 | 210 |
| The first hydrogenation fixed bed reactor | Copper-based catalyst | TG-45 | GC207 | TG-45 |
| | Average value (degrees centigrade) of hot-spot temperature of catalyst bed layer | 180 | 230 | 300 |
| | Flow of gasification furfural Kg/(Kg•h) | 0.05 | 1.0 | 2.0 |
| | Hydrogen and oil ratio | 4:1 | 15: 1 | 40:1 |
| Temperature of the heat exchanger | | 65 | 72.5 | 80 |
| The second hydrogenation catalytic reactor | Catalyst | HT-40 | RTH-2123E | RTH-2123E |
| | Average value (degrees centigrade) of hot-spot temperature of catalyst bed layer | 80 | 130 | 180 |
| | Flow of gasification furfural Kg/(Kg•h) | 0.05 | 1.0 | 2.0 |
| | Hydrogen and oil ratio | 4: 1 | 15: 1 | 40: 1 |
| Furfural conversion rate | | 99% | 95% | 99% |
| Purity of the target product (2-MeTHF) in crude products | | 86% | 93% | 90% |

| | | | | |
|---|---|---|---|---|
| Note: the hydrogen and oil ratio is the molar ratio between the hydrogen flow and the flow of the gasification furfural. | | | | |

It can see from the data of the embodiments 1-3 that, the catalyst of the disclosure is adopted to realize the continuous process of 2-MeTHF by using the furfural to implement gas-phase reaction in low pressure or ambient pressure, thus the traditional process of producing the 2-MeTHF which has high pressure, high investment and high risk is changed; and as the low-toxicity catalyst which is cheap and easy to get is adopted, the use of the high-toxicity or high-cost catalyst in the prior art is improved. In addition, by adopting the method of the disclosure to produce the 2-MeTHF, the flow of the gasification furfural, namely, the throughput of the raw material furfural in unit time is high, and the gasification furfural is easy to be separated from the impurities during the process, the conversion rate of the furfural can achieve 99%; the target product 2-MeTHF in the crude product has high purity, and by distilling and purifying the crude products, the purity of the final target product 2-MeTHF can be greater than 99%.

The above is only the preferred embodiment of the disclosure, but not intended to limit the disclosure; for those skilled in the field, the disclosure can have various changes and modifications. Any modifications, equivalent replacement and improvement implemented within the spirits and principle of the disclosure shall fall within the protection scope of the disclosure.

## Claims

1. A continuous processing method of 2-Methyltetrahydrofuran (2-MeTHF), **characterized by** comprising the steps as follows:
introducing gasification furfural and hydrogen into a first reaction zone and processing a first catalytic hydrogenation reaction;
introducing the gas output from the first reaction zone into a second reaction zone and processing a secondary catalytic hydrogenation reaction; and
condensing the gas output from the second reaction zone to obtain the 2-MeTHF;
wherein the first reaction zone is filled with a catalyst which is used for aldehyde group reduction, and the second reaction zone is filled with the catalyst which is used for aromatic saturation hydrogenation.

2. The method according to claim 1, **characterized in that**,
before introducing the gasification furfural and the hydrogen into the first reaction zone, further comprising a step of premixing the gasification furfural and the hydrogen.

3. The method according to claim 2, **characterized in that**,
before the secondary catalytic hydrogenation reaction, further comprising a step of implementing heat exchange for the gas output from the first reaction zone.

4. The method according to claim 1, **characterized in that**,
the catalyst used for aldehyde group reduction is the copper-based catalyst; and the catalyst used for the aromatic saturation hydrogenation is the nickel-based catalyst.

5. The method according to claim 4, **characterized in that**,
in the first reaction zone, the flow of the gasification furfural is 0.05-2.0Kg/(Kg•h) based on the weight of the copper-based catalyst, and preferably is 0.1-1.0Kg/(Kg•h);
in the second reaction zone, the flow of the gasification furfural is 0.05-2.0Kg/(Kg•h) based on the weight of nickel-based catalyst, and preferably is 0.1-1.0Kg/(Kg•h).

6. The method according to claim 5, **characterized in that**,
in the first reaction zone and the second reaction zone, the molar ratio of the hydrogen flow and the gasification furfural flow is greater than 4:1, preferably, the molar ratio is 5:1 to 100:1, and more preferably, the molar ratio is 7:1 to 40:1.

7. The method according to claim 1, **characterized in that**,
Providing a desulfurization catalyst and/or a carbon monoxide (CO) conversion catalyst to the first reaction zone and/or the second reaction zone.

8. The method according to claim 3, **characterized in that**,
the method comprises the steps:
pumping the liquid furfural into a gasification chamber via a charging pump, and gasifying the furfural to obtain the gasification furfural;
premixing the gasification furfural and the hydrogen in the gasification chamber, and inputting to a first hydrogenation fixed bed reactor to process the first catalytic hydrogenation reaction;
introducing the gas output from the first hydrogenation fixed bed reactor into a heat exchange device and processing heat exchange;
introducing the gas output from the heat exchange device into a second hydrogenation fixed bed reactor,and processing a secondary catalytic hydrogenation reaction; and
introducing the gas output from the second hydrogenation fixed bed reactor into a condensing unit for condensation to obtain the 2-MeTHF.

9. The method according to claim 8, **characterized in that**,
the hot-spot temperature of the catalyst bed layer of the first hydrogenation fixed bed reactor is 180 degrees centigrade to 300 degrees centigrade, and preferably is 180 degrees centigrade to 230 degrees centigrade;
the hot-spot temperature of the catalyst bed layer of the second hydrogenation fixed bed reactor is 80 degrees centigrade to 180 degrees centigrade, and preferably is 80 degrees centigrade to 130 degrees centigrade.

10. The method according to claim 8, **characterized in that**,
both the first hydrogenation fixed bed reactor and the second hydrogenation fixed bed reactor are tubular fixed bed reactors or multitubular fixed bed reactors.
